(19) **Europäisches Patentamt**
**European Patent Office**
**Office européen des brevets**

(11) **EP 4 284 764 B1**

(12) **EUROPÄISCHE PATENTSCHRIFT**

(45) Veröffentlichungstag und Bekanntmachung des Hinweises auf die Patenterteilung:
**12.06.2024   Patentblatt 2024/24**

(21) Anmeldenummer: **22714429.2**

(22) Anmeldetag: **15.03.2022**

(51) Internationale Patentklassifikation (IPC):
***C04B 2/10*** *(2006.01)*

(52) Gemeinsame Patentklassifikation (CPC):
**C04B 2/10;** Y02P 40/10; Y02P 40/18

(86) Internationale Anmeldenummer:
**PCT/EP2022/056594**

(87) Internationale Veröffentlichungsnummer:
**WO 2022/200112 (29.09.2022 Gazette 2022/39)**

(54) **REGELUNGSVERFAHREN ZUR STEUERUNG DER CALCINIERUNG VON TONEN FÜR DIE ZEMENTINDUSTRIE**

CONTROL PROCESS FOR CONTROLLING CALCINATION OF CLAYS FOR THE CEMENT INDUSTRY

PROCÉDÉ DE CONTRÔLE POUR LA RÉGULATION DE LA CALCINATION D'ARGILES POUR L'INDUSTRIE DU CIMENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.03.2021   DE 102021203044**
**26.03.2021   BE 202105231**

(43) Veröffentlichungstag der Anmeldung:
**06.12.2023   Patentblatt 2023/49**

(73) Patentinhaber:
• **thyssenkrupp Polysius GmbH**
**59269 Beckum (DE)**
• **thyssenkrupp AG**
**45143 Essen (DE)**

(72) Erfinder:
• **ENDERS, Michael**
**48143 Münster (DE)**

• **GRUND, Guido**
**59457 Werl (DE)**

(74) Vertreter: **thyssenkrupp Intellectual Property GmbH**
**ThyssenKrupp Allee 1**
**45143 Essen (DE)**

(56) Entgegenhaltungen:
WO-A1-01/32581

• **ROGER S. ALMENARES ET AL: "Industrial calcination of kaolinitic clays to make reactive pozzolans", CASE STUDIES IN CONSTRUCTION MATERIALS, Bd. 6, 1. Juni 2017 (2017-06-01), Seiten 225-232, XP055554255, ISSN: 2214-5095, DOI: 10.1016/j.cscm.2017.03.005**

**Beschreibung**

Die Erfindung betrifft ein Regelungsverfahren zur Optimierung der Produktqualität bei der Calcinierung von Tonen hinsichtlich der Reaktivität des Endprodukts.

Um die $CO_2$-Emissionsproblematik in den Griff zu bekommen plant man heutzutage knappe Kompositmaterialien wie Hüttensand und Flugasche durch calcinierten Ton als Bestandteil in Zementen zu ersetzen.

Aus der EP 3 218 320 B1 ist Verfahren zur Wärmebehandlung von natürlichen Tonen und/oder Zeolithen bekannt, wobei der Ton und/oder der Zeolith in der Calcinierzone in einem Flugstromcalcinator oder einer Wirbelschicht in einem Temperaturbereich von 350 °C bis 1050 °C unter reduzierenden Bedingungen calciniert wird, wobei während der Calcination unter reduzierenden Bedingungen wenigstens eine teilweise Reduktion von rötlich färbendem dreiwertigen Eisen auf zweiwertiges Eisen, insbesondere zu Magnetit ($FeO \cdot Fe_2O_3$), stattfindet.

Aus der US 9 458 059 B2 ist ein Verfahren zur Herstellung synthetischer Puzzolane mit gewünschter Farbcharakteristik mit einer Kühlung unter reduzierenden Bedingungen bekannt.

Aus der DE 10 2011 014 498 B4 ist ein Verfahren zur Herstellung eines Klinkerersatzstoffes für die Verwendung bei der Zementherstellung bekannt, wobei eine thermische Behandlung des Tones unter reduzierenden Bedingungen bei einer Temperatur von 600 °C bis 1.000 °C sowie eine Zwischenkühlung des Reduktionsproduktes unter Sauerstoffabschluss auf eine Temperatur < 300 °C erfolgt.

Aus der DE 10 2008 020 600 B4 ist ein Verfahren zur Wärmebehandlung feinkörniger mineralischer Feststoffe bekannt, wobei die Feststoffe durch einen Flash-Reaktor hindurchgeführt werden, in welchem sie bei einer Temperatur von 450 °C bis 1500 °C und einer Verweilzeit zwischen 0,5 und 20 Sekunden, mit heißen Gasen in Kontakt gebracht werden, und wobei die Feststoffe anschließend bei einer Temperatur von 500 °C bis 890 °C durch einen Verweilzeitreaktor geführt werden, aus welchem sie nach einer Verweilzeit von 1 bis 600 Minuten abgezogen werden.

Aus der US 2012 / 160 135 A1 ist ein Verfahren zur Herstellung synthetischer Puzzolane mit gewünschten Farbeigenschaften mit einer Kühlung unter reduzierender Atmosphäre bekannt.

Aus der EP 3 615 489 A2 ist ein Verfahren zur Herstellung von grauen synthetischen Puzzolanen mit einer ersten raschen Kühlung unter 600 °C bekannt.

Aus der WO 2015 / 039 198 A1 ist ein Verfahren zur Herstellung einer Zubereitung zur partiellen Substitution von Portland-Zement mit grauer Farbe bekannt.

Aus der WO 2016 / 041 717 A1 ist die Ermittlung der Reaktivität eines Produktes bekannt.

Während bei der Umsetzung von Kalk (grob vereinfacht $CaCO_3$) zu Zementklinker (grob vereinfacht CaO) pro Mol CaO auch ein Mol $CO_2$ erzeugt wird, wird beim Calcinieren von Tonen nur Wasser freigesetzt. Hierbei werden die Silikate beziehungsweise Aluminate im Ton umgesetzt und von einer nicht reaktiven Form in eine reaktive Form überführt, in der die Silicate beziehungsweise Aluminate nach Reaktion in einem basischen Milieu zunächst löslich sind und weiter mit dem bei der Reaktion von Klinker freigesetzten Calcium zu Calcium-Aluminium-Silikat-Hydrat Phasen (CASH) reagieren können. Werden diese Stoffe jedoch weiter oder länger erhitzt, so gehen diese in einen glasartigen und bei weiter erhöhten Temperaturen in inerte Mineralphasen (Spinelle, Mullit) über und verlieren somit ihren reaktiven Zustand wieder.

Betrachtet man ganz exemplarisch bei einer konstanten Verweilzeit die Calcination eines theoretischen Tones bei verschiedenen Temperaturen, so stellt man fest, dass erst bei einer Temperatur über beispielsweise 500 °C ein erstes Ansteigen der Reaktivität festzustellen ist, die über 600 °C deutlich gesteigert wird. Im Bereich von beispielsweise 700 °C bis 850 °C bildet sich ein annäherndes Plateau mit maximaler Reaktivität aus. Bei Temperaturen beispielsweise oberhalb von 900 °C ist dann ein rasches Absinken der Reaktivität festzustellen. Es ist daher wünschenswert, eine Calcination möglichst im Bereich dieses Plateaus (meist aus Kostengründen am unteren Ende) durchzuführen.

Tone und die durch Calcinierung hieraus herstellbaren Produkte lassen sich hinsichtlich ihrer reaktiven Eigenschaften in Bindemitteln jedoch nur sehr schwer analytisch charakterisieren. Chemische und mineralogische Analysen erlauben hinsichtlich der Reaktivität nur indirekte Rückschlüsse über Analogien. gut identifizieren und quantifizieren lassen sich mittels Röntgenbeugung gut kristalline Bestandteile wie Quarz ; diese sind jedoch gerade nicht reaktiv. Calcinierte Tone sind amorph und daher mit der Röntgenbeugung nur unzureichend zu quantifizieren. Daher wird teilweise versucht, mittels NIR Spektroskopie Informationen über die Zusammensetzung zu erhalten, was jedoch auch nur mangelhafte und unzureichend quantitative Informationen zur Reaktivität der calcinierten Tone gibt.

Dagegen gibt es jedoch Messmethoden, die die Reaktivität calcinierter Tone gut bestimmen könne, wie beispielsweise aus F. Avet et al., Development of a new rapid, relevant and reliable (R3) test method to evaluate the puzzolanic reactivity of calcined kaolinitic clay, Cement and Concrete Research 85 (2016) 1-11, Elsevier bekannt. Diese Methode ist, wie unter Punkt 3 Methoden, Unterpunkt 3.1 auf eine Messzeit von 7 Tagen ausgelegt. Ein solcher Zeitraum macht eine aktive Regelung einer Produktionsanlage unmöglich.

Aus der WO 2016 / 096 911 A1 ist eine kalorimetrische Messeinrichtung zur Ermittlung der Reaktivität bekannt.

Aus der ROGER S. ALMENARES ET AL: "Industrial calcination of kaolinitic clays to make reactive pozzolans", CASE STUDIES IN CONSTRUCTION MATERIALS, Bd. 6, 1. Juni 2017 (2017-06-01), Seiten 225-232, XP055554255, ISSN: 2214-5095, DOI: 10.1016/j.cscm.2017.03.005 ist die industrielle Kalzination von kaolinitischem Ton zur Herstellung reaktiver Puzzolane bekannt.

Aus der WO 01/32581 A1 ist ein Kontrollsystem für eine Klinkeranlage bekannt.

Aufgabe der Erfindung ist es, eine neuartige Regelung zu schaffen, die nicht auf andere Stoffparameter, sondern auf die tatsächliche Reaktivität abzielt und als zeitnahe Rückkopplung und somit aktive Regelung verwendbar ist.

Gelöst wird diese Aufgabe durch das Regelungsverfahren mit den in Anspruch 1 angegebenen Merkmalen. Vorteilhafte Weiterbildungen ergeben sich aus den Unteransprüchen, der nachfolgenden Beschreibung sowie den Zeichnungen.

Das erfindungsgemäße Regelungsverfahren dient zur Regelung eines Herstellungsverfahrens für calcinierte Tone mit einem Calcinator. Derartige Verfahren sind umfänglich aus dem Stand der Technik bekannt. Das Regelungsverfahren ist vom konkreten Herstellungsverfahren unabhängig und lässt sich auf alle entsprechenden Verfahrensvarianten für die Herstellung calcinierter Tone anwenden. Das Regelungsverfahren weist die folgenden Schritte auf:

a) Erfassen wenigstens einer Temperatur im Calcinator und optional einer ersten mittleren Verweilzeit des Tones im Calcinator,

b) Entnehmen wenigstens einer Probe des calcinierten Tones,

c) Herstellen einer reproduzierbaren Größenverteilung der Probe,

d) Einstellen der Probenmasse auf eine vordefinierte Probenmasse,

e) Temperieren der Probe auf eine erste Messtemperatur,

f) Vermengen der Probe mit einer Lauge, welche bevorzugt ebenfalls auf eine erste Messtemperatur temperiert ist,

g) Zeitliches Erfassen der von der Probe-Laugen-Mischung erzeugten Energie bei konstanter erster Messtemperatur für einen ersten Zeitraum,

h) Quantitatives Auswerten des in Schritt g) erfassten Zeit-Energie-Verlaufs für die erste exotherme Reaktion und Ermitteln der durch die Probe freigesetzten Energiemenge für die erste exotherme Reaktion,

i) Korrelation der in Schritt h) erfassten Energiemenge mit der in Schritt a) erfassten Temperatur und Verweilzeit sowie Vergleich mit vorher erfassten Wertekombinationen aus Energiemenge, Temperatur und Verweilzeit,

j) Aktives Regeln der Temperatur und/oder der Verweilzeit im Calcinator in Richtung der Erhöhung der für eine weitere Probe zu erwartende Energiemenge.

In Schritt a) wird die Temperatur im Calcinator erfasst. Da die Temperatur im Calcinator an verschiedenen Stellen unterschiedlich ist, ist für das Verfahren vor allem wesentlich, dass die Temperatur immer in gleicher Weise, also beispielsweise mit dem gleichen Messfühler an der gleichen Stelle gemessen wird. Zusätzlich können natürlich auch an weiteren Stellen weitere Temperaturen erfasst und berücksichtigt werden, um ein genaueres Bild zu ergeben, beispielsweise, wenn durch die Verwendung sehr unterschiedlicher Brennstoff mit sehr unterschiedlichen Brennverhalten unterschiedliche Temperaturverläufe erzielt werden würden.

In Schritt a) kann weiter auch die mittlere Verweilzeit erfasst werden, sofern diese gezielt, insbesondere getrennt von der Temperatur im Calcinator regelbar ist. Ist die Verweilzeit nicht einstellbar, insbesondere nicht veränderbar oder nur durch eine Temperaturänderung veränderbar, so ist eine zusätzliche Erfassung der Verweilzeit neben der Temperatur im Calcinator nicht nötig.

Das Entnehmen der Probe des calcinierten Tones in Schritt b) kann bevorzugt automatisiert erfolgen. Bevorzugt ist die automatisierte Probenentnahme an eine automatisierte Probenvorbereitung angebunden. Die entnommene Probe kann deutlich größer als die vordefinierte Probenmasse sein, um zusätzlich zur Durchführung des erfindungsgemäßen Verfahren auch gleichzeitig ein Rückstellmuster aus der genommenen Probe entnehmen zu können.

Die SI-Einheit für die Masse ist das Kilogramm (kg). Die Probenmasse kann aber bevorzugt in Gramm (g) oder Milligramm (mg) gemessen werden. Natürlich sind, insbesondere in beispielsweise angelsächsisch geprägten Regionen, andere Einheiten möglich, beispielsweise Pfund und Unzen. Der Fachmann ist in der Lage, diese ineinander umzurechnen.

Das Einstellen der Probenmasse auf eine vordefinierte Probenmasse in Schritt d) erfolgt dadurch, dass die Probe mengenmäßig auf eine vorgegebene Größe reduziert wird. Da zum Beispiel in Schritt c) Verluste auftreten, wird die in Schritt b) genommene Probe größer sein, als die in den Schritte e), f) und g) verwendete Probe. Wird hierfür beispielsweise eine Probe von exakt 10,0 g (beispielhafte vorgegebene Größe) genommen, so kann beispielsweise in Schritt b) eine Probe von ungefähr 25 g genommen werden, die in Schritt c) beispielsweise vermahlen werden. In Schritt d) wird dann daraus eine Teilprobe von genau 10,0 g genommen, der Rest der Probe verworfen. Die vorgegebene Größe hängt somit ausschließlich von den Anforderungen der für den Schritt g) verwendeten Vorrichtung ab.

Wesentlich ist, dass in Schritt h) ausschließlich der sogenannte Initialpeak, die erste messbare exotherme Reaktion erfasst wird. Betrachtet man die von der Probe abgegebene Wärme über die Zeit, so stellt man fest, dass ein oder mehrere exotherme Reaktionen nacheinander erfolgen, es also mehrere Maxima in der Auftragung Energie gegen die Zeit gibt. Während die weiteren Schritte (zweites und alle weiteren Maxima) über einen Zeitraum von bis zu sieben Tagen ablaufen und ein exaktes Abbild über die Reaktivität und das Abbindeverhalten geben, wird erfindungsgemäß ausschließlich dieser erste Reaktionsschritt (das erste Maximum in der Auftragung der abgegebenen Wärme als Funktion der Zeit) betrachtet. Es hat sich gezeigt, dass die hier freigesetzte Wärmemenge bestimmt zum Beispiel als Maximum des Peaks, Integral, Fläche unter der Kurve, kumulative abgegebene Wärme) eine sehr gute Korrelation zur Reaktivität des calcinierten Tones aufweist. Bisher wurde dieser Bereich regelmäßig nicht beachtet, da zum einen auch eine rasche händische Probenpräparation mehrere Minuten andauert, zum anderen durch das Öffnen der Messzelle eine thermische Störung eingebracht wird, die eine Verifizierung der sogenannten Basislinie erfordert. Beide Effekte stören durch Variation in Dauer und Intensität den Anfangsbereich der Messung, welcher bereits den initialen Peak aufweist. Daher ist erfindungswesentlich, dass eine für alle Proben vergleichbare Größenverteilung eingestellt wird. Hierbei ist weniger relevant eine bestimmte mittlere Größe oder eine bestimmte Breite der Größenverteilung einzustellen und die genaue Kenntnis der Größenverteilung ist auch nicht notwendig. Es reicht lediglich, wenn eine Kontinuität über alle Messung erreicht werden kann. Bevorzugt erfolgt das Herstellen einer reproduzierbaren Größenverteilung der Probe in Schritt c) durch Mahlen. Es hat sich herausgestellt, dass sich bei immer gleicher Präparation in der gleichen Mühle für die gleiche Zeit auch bei gewissen Schwankungen in der ursprünglichen Probe eine ausreichend einheitliche Größenverteilung einstellt. Besonders bevorzugt erfolgt die Probenpräparation daher auch automatisiert, da hierdurch die Gleichmäßigkeit weiter gesteigert werden kann.

Die erfindungsgemäße Verwendung des Initialpeaks ermöglicht überhaupt nur das erfindungsgemäße Regelungsverfahren. Natürlich vergeht zwischen Schritt b) und Schritt j) eine gewisse Zeit, typischerweise ein bis zwei Stunden. Damit ist natürlich verbunden, dass die Regelung erst zeitversetzt erfolgt. Da aber die Produktion typischerweise kontinuierlich betrieben wird und die Schwankungen meist eher geringer beziehungsweise langfristiger sind, beispielsweise, weil ein Rohstoff aus einer anderen Halde zugeführt ist und etwas andere Eigenschaften aufweist, kann eine auf der Zeitskale der Produktion dennoch ausreichend zeitnahe Nachregulierung erfolgen. Somit kann der erfindungsgemäße Nutzen auch realisiert werden, wenn durch das Verfahren eine entsprechende Differenz zwischen Probennahme in Schritt b) und Regelung in Schritt j) liegt. Der große Vorteil gegenüber den herkömmlichen Verfahren liegt also in einem Zeitgewinn von bisher zwei bis sieben Tagen auf lediglich zwei Stunden durch die Einschränkung auf den Initialpeak anstelle des vollständigen Abbindeverhaltens. Aus dem Stand der Technik ist eben bekannt, dass man den Initialpeak gerade nicht beachtet, da dieser üblicherweise durch Probenvorbereitung und dergleichen stärker beeinflusst ist als die späteren Vorgänge im Abbindeverhalten. Dass aber dennoch der Initialpeak repräsentativ für das gesamte Abbindeverhalten ist, wenn man die Probenpräparation in der erfindungsgemäßen Weise durchführt ist das Neue an diesem Regelungsverfahren.

Ebenso hat sich als sehr vorteilhaft herausgestellt die Probe auf die erste Messtemperatur einzustellen. Dieses kann neben dem Schritt e) auch bereits früher erfolgen. Während eines Mahlvorganges wird jedoch regelmäßig vergleichsweise viel Energie eingetragen, sodass es zu einer Erwärmung der Probe kommt. Besonders bevorzugt ist auch die in Schritt f) zugegebene Lauge bereits vor der Zugabe auf genau die erste Messtemperatur temperiert. Je näher die in die Messung eingebrachte Probe-Laugen-Mischung der ersten Messtemperatur kommt, umso exakter sind die Messergebnisse für den Initialpeak, welcher in Schritt g) erfasst und in Schritt h) ausgewertet wird. Die erste Messtemperatur wird vorzugsweise mit einer Genauigkeit von 0,001 K bis 0,3 K eingestellt, besonders bevorzugt mit einer Genauigkeit von 0,001 K bis 0,1 K, weiter bevorzugt von 0,01 K bis 0,1 K.

Besonders bevorzugt wird die Messung so schnell wie möglich nach der Zugabe der Lauge zur Probe begonnen. Dieses wird zum einen dadurch erleichtert, wenn bereits beide Komponenten vor dem Zusammenbringen bereits die gleiche erste Messtemperatur aufweisen. Zum anderen erfolgt das Vermengen in Schritt f) mit möglichst geringem Energieeintrag, beispielsweise mittels Ultraschall oder einem intensiven Mischer auf Basis von Pressluft. Es wird ein kurzes inniges Vermischen bevorzugt, um anschließend die Probe ohne weitere Temperierungszeiten vermessen zu können, da die Reaktion zwischen dem calcinierten Ton und der Lauge unmittelbar mit der Zugabe beginnt. Elektrische oder mechanische Mischvorrichtungen sind nur dann geeignet, wenn eine Einbringung von Wärme deutlich unterhalb der bei der Reaktion freigesetzten Wärme sichergestellt werden kann.

Diese Fokussierung auf eine Vergleichmäßigung und thermische Stabilisierung der Probe vor der Laugenzugabe und dem Messbeginn führt dazu, dass bereits der Initialpeak aussagekräftig ausgewertet werden kann.

In Schritt i) erfolgt eine Korrelation, um daraus eine Regelungsaussage für das aktive Regeln in Schritt j) zu bekommen. Hierbei ist eine Korrelation zwischen der in Schritt h) ermittelten Energiemenge mit der in Schritt a) erfassten Temperatur notwendig, da die Reaktivität und damit die durch den Initialpeak erfasste Energiemenge keine monoton steigende oder fallende Abhängigkeit von der Temperatur aufweist, sondern ein Maximum im Optimum erreicht. Stellt man nun im Vergleich mit vorherigen Messwerten eine abfallende Energiemenge und damit ein Abfall der Reaktivität des Produktes fest, so würde sich alleine aus dieser Information noch nicht ergeben, ob die Temperatur zur Wiedererlangung des Optimums gesteigert oder gesenkt werden muss. Dieses ergibt sich erst aus der Korrelation mit den jeweiligen Temperaturwerten. Erschwerend kommt hinzu, dass auch die Edukte, die eingesetzten Tone, unterschiedlich ausfallen, sodass gegebenenfalls nach einer Korrektur in einem nächsten Zyklus festgestellt wird, dass die Korrektur in die andere Richtung hätte erfolgen müssen und dann entsprechend korrigiert wird. Hierbei muss nicht zwingend auf das Maximum geregelt werden, zumal das Maximum Bestandteil eines Plateaus ist, sodass eine Regelung eher darauf abzielt, sich innerhalb des Plateaus zu bewegen. Hierbei kann zur Regelung auch der Energiebedarf der Anlage hinzugezogen werden, sodass beispielsweise zur Verminderung der Heizkosten und der damit verbundenen Emissionen eher am unteren (kälteren) Ende des Plateaus produziert wird. Ebenso können weitere Parameter als zusätzliche Regelkriterien zur Optimierung innerhalb des Plateaus herangezogen werden.

In einer weiteren Ausführungsform der Erfindung wird als Lauge in Schritt f) eine Alkalihydroxidlösung mit einem pH zwischen 9 und 15, bevorzugt zwischen 10,25 und 15, weiter bevorzugt zwischen 12 und 14,6, ausgewählt. Besonders bevorzugt ist die Lauge eine wässrige Natronlauge mit einer Konzentration zwischen $0{,}1 \,^{mol}/_{l}$ und $1 \,^{mol}/_{l}$. Beispielsweise wird eine Natronlauge mit einer Konzentration von $1 \,^{mol}/_{l}$ verwendet (pH ~ 14).

In einer weiteren Ausführungsform der Erfindung entspricht die Zugabemasse der Lauge dem, 0,5fachen bis 5fachen, bevorzugt dem 1fachen bis 5fachen, weiter bevorzugt den 2fachen bis 3fachen, der Masse der Probe. Es ergibt sich bei gleicher Masse somit ein Gewichtsverhältnis von 1:1 für Probe zu Lauge und bei 5facher Masse an Lauge ein Gewichtsverhältnis von 1:5 für Probe zu Lauge. Werden also beispielsweise 5 g als vordefinierte Probenmasse eingesetzt, so werden 5 bis 15 g einer beispielsweise $1 \,^{mol}/_{l}$ wässrigen Natriumhydroxidlösung, beispielsweise 10 g zu der Probe gegeben und mit dieser vermengt. Hierdurch wird zum einen eine Ausreichende Menge an Hydroxylionen zur Verfügung gestellt. Außerdem steht auch genügend Volumen zur Verfügung, um die Probenoberfläche vollständig zu benetzen und auch alle Poren zu füllen. Die zugegebene Laugenmenge wird dabei bevorzugt automatisiert im gleichen Verhältnis an geringfügig durch eventuelle Einwaageunterschiede an die, anhand einer festgestellten vom Sollwert abweichenden Probenmengen, angepasst. Somit wird ein konstantes Verhältnis zwischen Probe und Lauge sichergestellt. Dieses erleichtert auch eine anschließende Normierung der erhaltenen Energie des Initialpeaks auf die exakte Einwaage.

In einer weiteren Ausführungsform der Erfindung werden als Lauge in Schritt f) ein Laugenbildner und Wasser zugegeben, wobei der Laugenbildner und das Wasser in Reaktion miteinander eine Lösung mit einem pH zwischen 9 und 15, bevorzugt zwischen 10,25 und 15, weiter bevorzugt zwischen 12 und 14,6, erzeugen. Beispielsweise und insbesondere ist der Laugenbildner ausgewählt aus der Gruppe umfassend Alkalihydroxid, Alkalioxid, Erdalkalihydroxid, Erdalkalioxid sowie Stoffe, Gemische oder Zusammensetzungen, die diese enthalten oder bei Reaktion mit Wasser freisetzen.

Insbesondere kann das Erdalkalioxid Calciumoxid sein, besonders bevorzugt ist eine Zusammensetzung, welche Calciumoxid enthält oder freisetzt, ein Zementklinker. Beispielsweise kann eine Probe von 2.5 g calciniertem Ton mit 2.5 g Zementklinker und 5 g Wasser gemischt werden. Hierdurch kann die Reaktivität des calcinierten Tones in einer besonders anwendungsnahen Umgebung untersucht werden.

In einer weiteren Ausführungsform der Erfindung erfolgen die Schritte c) bis g) automatisiert in einer auf die erste Messtemperatur klimatisierten Umgebung. Hierdurch kann die Probe relativ schnell thermisch stabilisiert werden.

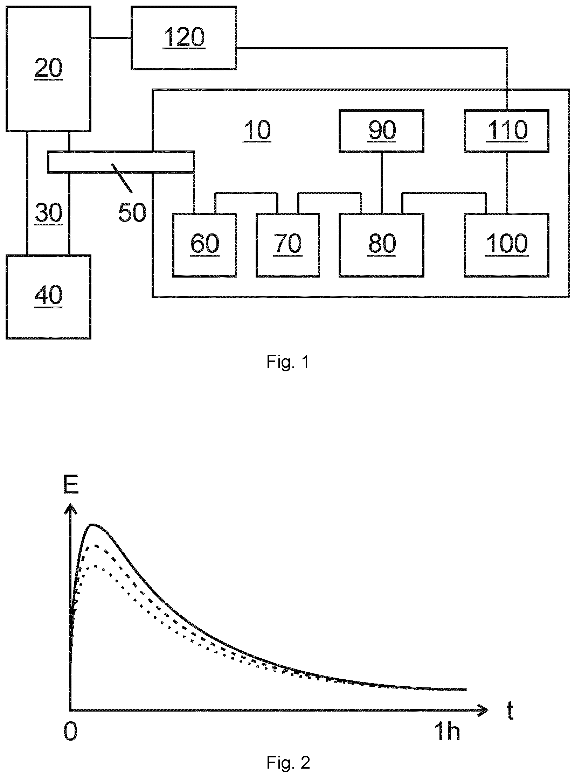

In einer weiteren Ausführungsform der Erfindung wird die Probenmasse in Schritt d) auf wenigstens 2 % genau, bevorzugt wenigstens 0,5 % genau, weiter bevorzugt wenigstens 0,1 % genau, besonders bevorzugt auf wenigstens 0,02 % genau, auf die vorgegebene Probenmasse eingestellt. Je genauer die Probenmasse auf die vorgegebene Probenmasse eingestellt wird, umso geringer ist der Messfehler auf die im Initialpeak freigesetzte Energiemenge. Wird die zugegebene Laugenmenge auf die exakte Einwaage angepasst, so ist eine anschließende Normierung ebenfalls leichter möglich. Erfindungsgemäß erfolgt die quantitative Auswertung des in Schritt g) erfassten Zeit-Energie-Verlaufs für die erste exotherme Reaktion für den Zeitraum von der ersten Minute nach der Zugabe der Lauge bis zu 120. Minute, weiter bevorzugt von der zweiten Minute nach der Zugabe der Lauge bis zur 70. Minute. Der Zeitraum von der Zugabe der Anregerflüssigkeit bis zum Messbeginn kann als Nullwert herangezogen werden. Bevorzugt werden somit lediglich die ersten 30 Sekunden bis zu maximal zwei Minuten nicht betrachtet, die den Zeitbedarf für die Probenmischung und den Transport der Probe gemischt mit der Lauge in das Analysegerät bis zur ersten Messung einer Temperaturänderung der Proben-Laugen-Mischung umfassen. Somit wird nicht für die volle Reaktionszeit von etwa 7 Tagen betrachtet, sondern nur für den Zeitraum von rund einer Stunde bis zu zwei Stunden nach Zugabe der Lauge. Dieses ermöglicht auch eine zeitnahe Rückkopplung zum Herstellungsverfahren. Dieses Zeitfenster hat sich als aussagekräftig und dennoch kurz genug erwiesen. Natürlich wäre es vorteilhaft, wenn Laugenzugabe und Mischvorgang bereits innerhalb der Erfassung erfolgen könnte, sodass auch der unmittelbare Beginn, also der Zeitraum der ersten zwei Minuten mit erfassbar wäre. Wie in Fig. 2 dargestellt, ist der Initialpeak typischerweise nach 10 bis 20 min bereits am abfallen. Durch den starken Abfall wird nach über zwei Stunden, bevorzugt nach einer Stunde bereits kein deutlicher Mehrwert mehr gewonnen, die durch Integration unter der Messkurve gemessene Fläche vergrößert sich nicht mehr deutlich, oft sogar durch das Rauschen nicht erkennbar. Durch die Verkürzung des Messzeit kann aber die Rückkopplung verbessert werden, wofür eine geringfüge Verschlechterung der Auswertung durch gegebenenfalls nicht vollständiges Erfassen des letzten Endes des Initialpeaks leicht in Kauf genommen werden kann und sogar bevorzugt ist.

In einer weiteren Ausführungsform der Erfindung wird die erste Messtemperatur im Bereich von 20 °C bis 40 °C gewählt. Beispielsweise und bevorzugt wird in Europa 20 °C, 21 °C oder 22 °C gewählt, während in wärmeren Regionen, beispielsweise den Subtropen, eine Temperatur von beispielsweise 27 °C gewählt wird. Eine höhere Temperatur, zum Beispiel 38 °C bis 40 °C, kann gewählt werden, um die Reaktionsgeschwindigkeit zu erhöhen und damit das Zeitintervall für den Initialpeak zu verkürzen. Eine höhere Temperatur ist möglich, benötigt jedoch einen höheren Heizaufwand und damit laufende Kosten.

In einer weiteren Ausführungsform der Erfindung wird neben der ersten Messtemperatur das Verfahren alternierend oder parallel mit einer zweiten Messtemperatur durchgeführt. Beispielsweise wird 20 °C als erste Messtemperatur und 40 °C als zweite Messtemperatur gewählt. Hierdurch kann eine zusätzliche Information über den thermischen Einfluss der Temperatur auf die Reaktivität über die Aktivierungsenergie ermittelt werden.

In einer weiteren Ausführungsform der Erfindung wird die vordefinierte Probenmasse zwischen 1 g und 200 g, bevorzugt zwischen 2 g und 20 g, ausgewählt.

In einer weiteren Ausführungsform der Erfindung wird in Schritt a) zusätzlich erfasst, aus welchen Eduktchargen in welchem Mischungsverhältnis der Ton der Calcination zugeführt wird. Beispielsweise kann eine Eduktcharge eine Halde oder ein ähnlicher Lagerbereich mit einem im Regelfall einheitlichem Material sein. In Schritt i) wird zusätzlich die Information aus welchen Eduktchargen in welchem Mischungsverhältnis der Ton der Calcination zugeführt wurde, verwendet. Insbesondere wird berücksichtigt, welche Eduktchargen bei einem höheren Mischungsanteil zu einer höheren oder geringeren Reaktivität führen. Hierdurch kann die gewünschte Zielreaktivität für das Produkt mit den geringsten Eduktreaktivitäten und damit dem günstigsten Ausgangsmaterial hergestellt werden. Zur Regelung in Schritt j) wird somit zusätzlich die Auswahl und das Mischungsverhältnis zwischen den Eduktchargen berücksichtigt.

In einer weiteren Ausführungsform der Erfindung weist das Regelungsverfahren zusätzlich zur Charakterisierung jeder Produktcharge ein Chargencharakterisierungsverfahren auf, wobei das Chargencharakterisierungsverfahren mit den folgenden Schritten durchgeführt wird:

A) Erwärmen einer Chargenprobe auf eine Chargentemperatur von 600 °C bis 1000 °C, bevorzugt 600 °C bis 950 °C für eine Chargenzeit von 1 s bis 60 min, bevorzugt von 30 s bis 20 min, besonders bevorzugt von 30 s bis 5 min,

B) Herstellen einer reproduzierbaren Größenverteilung der Chargenprobe,

C) Einstellen der Chargenprobenmasse auf eine vordefinierte Probenmasse,

D) Temperieren der Chargenprobe auf eine erste Messtemperatur,

E) Vermengen der Chargenprobe mit einer Lauge,

F) Zeitliches Erfassen der von der Chargenprobe-Laugen-Mischung erzeugten Energie bei konstanter erster Messtemperatur für einen ersten Zeitraum,

G) Quantitatives Auswerten des in Schritt F) erfassten Zeit-Energie-Verlaufs für die erste exotherme Reaktion und Ermitteln der durch die Chargenprobe freigesetzten Energiemenge für die erste exotherme Reaktion.

In Schritt A) wird somit eine modellhafte Calcination der Chargenprobe vorgenommen. Dieses erfolgt bevorzugt unter stark kontrollierten Laborbedingungen und somit unter klar reproduzierbaren Bedingungen. Diese so vorbereitete Chargenprobe wird anschließend wie die normale Probe aus dem Herstellungsverfahren behandelt. Somit wird Schritt B) identisch ausgeführt wie Schritt c), Schritt C) wird identisch wie Schritt d) ausgeführt, Schritt D) wird identisch wie Schritt e) ausgeführt, Schritt E) wird identisch wie Schritt f) ausgeführt, Schritt F) wird identisch mit Schritt g) ausgeführt, Schritt G) wird identisch wie Schritt h)ausgeführt. Dieses gilt insbesondere auch für Weiterbildungen der Verfahrensschritte des Regelungsverfahrens, die entsprechend im Chargencharakterisierungsverfahren angewendet werden.

In Schritt A) kann die Präparation beispielsweise in einem Tiegel in einem Muffelofen erfolgen. Für ein derartiges Verfahren ist oftmals ein Zeitraum von 15 min bis 1 h notwendig. Vorteilhaft wird die Präparation jedoch direkt auf einem heißen und beheizten Metall erfolgen, auf welchem die Probe gleichzeitig durch die Heizeinrichtung transportiert wird. Durch den direkteren Kontakt und den damit verbesserten Wärmeübergang sind deutlich kürzere Zeiten, insbesondere zwischen 1 min und 5 min möglich.

Insbesondere kann hierdurch auch bereits vor dem Verwenden neuer Ausgangsstoffe eine erste Datengrundlage für das Regelungsverfahren in Schritt i) verwendet werden, bevor Daten aus dem Herstellungsverfahren vorliegen.

In einer weiteren Ausführungsform der Erfindung wird das in Schritt A) für alle Chargenproben eine identische erste Chargentemperatur und eine identische erste Chargenzeit gewählt.

In einer weiteren Ausführungsform der Erfindung wird das Chargencharakterisierungsverfahren mit einer zweiten Chargentemperatur und eine zweiten Chargenzeit wiederholt. Beispielsweise ist die erste Chargentemperatur 750 °C und die zweite Chargentemperatur 850 °C. Um die Genauigkeit zu erhöhen sind natürlich auch weitere Chargentemperaturen und/oder Chargenzeiten denkbar.

Nachfolgend ist das erfindungsgemäße Regelungsverfahren anhand eines in den Zeichnungen dargestellten Ausführungsbeispiels näher erläutert.

Fig. 1 Schematische Darstellung einer Vorrichtung zur Durchführung des Verfahrens

Fig. 2 Initialpeak

Fig. 3 Reaktivität in Abhängigkeit der Calcinationstemperatur

Fig. 4 Automatisierte Analysevorrichtung in der Draufsicht

In Fig. 1 ist eine Vorrichtung gezeigt, in der das erfindungsgemäße Regelungsverfahren eingesetzt wird. Anhand der Vorrichtung soll der Ablauf des Verfahrens erläutert werden. In einem Calcinator 20 wir ein Ton calciniert und der calcinierte Ton über eine Produktentnahme 30 aus dem Calcinator 20 in ein Produktlager 40 überführt. Im Bereich der Produktentnahme 30 entnimmt eine Probenentnahme 50 eine Probe des calcinierten Tones und überführt die Probe in eine Analysevorrichtung 10, welche ein klimatisiertes Gehäuse aufweist. Beispielsweise wird als erste Messtemperatur 21 °C gewählt und der Innenraum der Analysevorrichtung 10 wird auf 21 °C temperiert. Es hat sich gezeigt, dass die Regelgenauigkeit bevorzugt auf 0.1 K oder besser bis auf 0.05 K genau erforderlich ist, solange an den Messwerten keine mathematische Korrekturfunktion bei Temperaturabweichungen angewandt werden soll. Die Probe wird zunächst mit Hilfe einer Mühle 60, beispielsweise einer Kugelmühle mit einer Probenkammer und Mahlkugeln aus Achat, für eine vordefinierte Zeit, beispielsweise 2 min, gemahlen. Hierdurch wird eine für alle Proben reproduzierbare Größenverteilung eingestellt. Anschließend wird mittels einer Waage 70 eine vordefinierte Probenmasse eingewogen, beispielsweise 5 g $\pm$ 0,02 g, und anschließend wird die Probe mit beispielsweise 10 g $\pm$ 0,02 g einer $1\,^{mol}/_l$ wässrigen Natriumhydroxidlösung versetzt und in einer Mischvorrichtung kurz und intensiv vermischt. Anschließend wird die Probe-Laugen-Mischung in ein isothermes Kalorimeter 100 eingebracht und die durch die Reaktion entstehenden Energieflüsse gegen die Zeit erfasst. In einer Analysenelektronik 110 wird die Gesamtenergie des Initialpeaks, der innerhalb der erste Stunde in einem ersten Reaktionsschritt freigesetzte Energie ausgewertet. Zum Zeitpunkt der Entnahme der Probe durch die Probenentnahme 50 erfasst die Steuerelektronik 120 die Temperatur des Calcinators 20 und korreliert diese Information mit der durch die Analyseelektronik 110 ermittelte Energie des Initialpeaks der Probe (zum Beispiel Maximum des Peaks oder Integral der Peakfläche oder die zu einem Zeitpunkt kumulativ freigesetzte Wärme). Durch den Vergleich mit vorhergehenden Messungen kann die Steuerelektronik 120 dann feststellen, ob eine Temperaturänderung des Calcinators 20 zur Verbesserung der Reaktivität des calcinierten Tons sinnvoll ist. Anschließend kann die Steuerelektronik 120 entweder den Calcinator 20 direkt ansteuern, beispielsweise durch Veränderung der Brennstoffzuführungsgeschwindigkeit oder die Steuerelektronik 120 kann eine solche Veränderung dem Operator der Anlage vorschlagen.

Fig. 2 zeigt ganz schematisch den in einem isothermen Kalorimeter gemessene Energie als Funktion der Zeit von drei Proben, welche bei unterschiedlichen Herstellungsbedingungen hergestellt wurden. Gezeigt ist der Initialpeak innerhalb der ersten Stunde der Reaktion bei der Umsetzung von calciniertem Ton mit der dreifachen Menge an $1\,^{mol}/_l$ NaOH-Lösung. Das Integral unter der Kurve entspricht der bei der Hydrolyse freigesetzten Energie und ist somit proportional zur Anzahl der reaktiven Zentren. Es hat sich gezeigt, dass die in Fig. 2 gezeigte Probe mit durchgezogener Linie auch bei einer Messzeit von mehreren Tagen die höchste Reaktivität aufweist, gefolgt von der Probe mit der gestrichelten Linie. Die Probe mit der gepunkteten Linie weist sowohl beim Initialpeak als auch bei der Messung über mehrere Tage die geringste Reaktivität auf. Daher kann die Fläche unter dem Initialpeak einfach durch Integration über die erste Stunde ermittelt werden und diese Fläche ist ein gutes direktes Maß für die Reaktivität der Probe. Das Maximum liegt in einem Bereich von 2 min bis zu 10 min, nach etwa 30 min ist Messwert schon unter den Startwert gefallen.

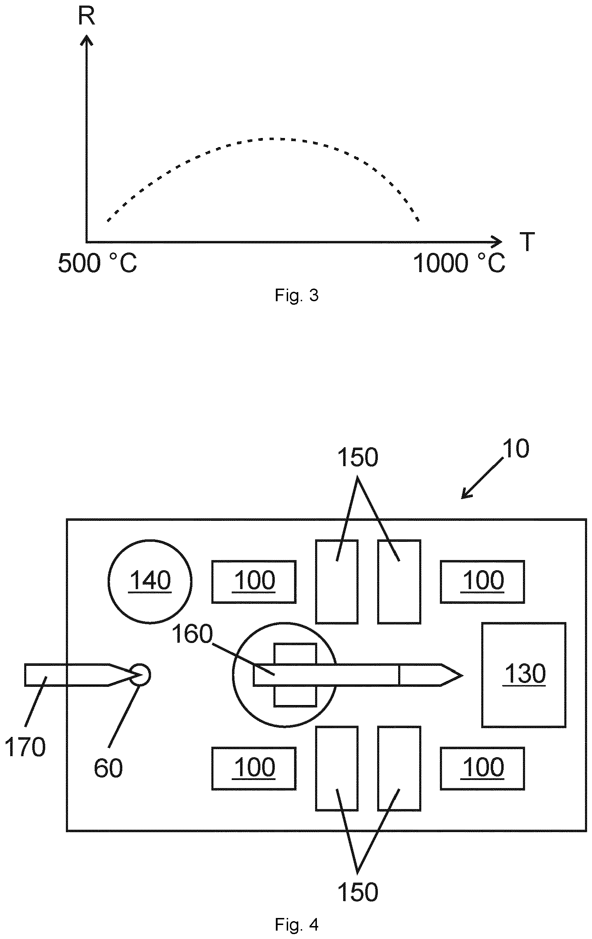

In Fig. 3 ist eine stark vereinfachte Auftragung der Reaktivität R (proportional zur gemessenen Energie) in Abhängigkeit der Temperatur im Calcinator 20 aufgetragen. Unter 600 °C ist eine sehr geringe Reaktivität, über 950 °C sinkt die Reaktivität sehr rasch aufgrund von Verglasung oder der teilweisen Kristallisation des Produktes. Ziel des Regelungsverfahrens ist es möglichst im Bereich des Maximums dieser Kurve den Herstellungsprozess zu fahren.

In Fig. 4 ist eine beispielhafte automatisierte Analysevorrichtung 10 in der Draufsicht gezeigt. Über eine Probenzuführung 170 wird die Probe beispielsweise über eine Vibrationsförderrinne in die Analysevorrichtung 10 eingebracht und bevorzugt dabei auch gewogen beziehungsweise portioniert. Hierzu hat zuvor ein Roboter 160 einen Probenbehälter aus dem Probenbehältervorrat 140 entnommen und in die Mühle 60 eingesetzt. In Probe wird in den Probenbehälter eingeführt und in dem Probenbehälter in der Mühle 60 gemahlen. Von dort transportiert der Roboter 160 den Probenbehälter in einen Stellplatz in einem Lager- und Temperierbereich 150. Beispielsweise weist ein Lager- und Temperierbereich 150 etwa 25 Stellplätze auf, insgesamt wären das im gezeigten Beispiel dann etwa 100 Stellplätze, an denen die Proben nach dem Mahlen und vor der Zugabe einer Lage temperiert werden können. Beispielsweise und bevorzugt wird der Lager- und Temperierbereich 150 daher von einem Wärmetauscherfluid durchströmt, um eine möglichst schnelle und gute Temperierung zu ermöglichen. Vorzugsweise stellt der Lager- und Temperierbereich 150 die Probe auf die erste Messtemperatur ± 0,1 K ein. Danach wird der Probenbehälter mit der Probe vom Roboter 160 in die Laugenzugabevorrichtung 130 verbracht. Die Laugenzugabevorrichtung 130 weist vorzugsweise einen Laugenvorrat 90 mit auf die erste Messtemperatur temperierte Lauge, beispielsweise

$1 \, {}^{mol}/_l$ Natronlauge, auf. Die Lauge wird entsprechend der exakten Einwaage der Probe zugegeben, beispielsweise die dreifache Menge, also 15 g Lauge auf 5 g Probe. In der Laugenzugabevorrichtung 130 ist auch eine Mischvorrichtung 80 angeordnet, vorzugsweise in Form einer Druckluftzufuhr. Weiter weist die Laugenzugabevorrichtung 130 eine Kamera zur optischen Erfassung der Probe auf. Dieses dient dazu, um zu erkennen, ob Tropfen aus Lauge und Probe im oberen Wandbereich des Probenbehälters anhaften. Ist dieses der Fall wird die dort freigesetzte Wärmemenge nicht zuverlässig erfasst, was zu Messfehlern führen könnte. Von Hier wird der Probenbehälter in ein isothermes Kalorimeter 100 verbracht. Beispielsweise und bevorzugt weist jedes isotherme Kalorimeter 8 Messplätze auf. Nach dem Einbringen des Probenbehälters in das isotherme Kalorimeter wird der Messplatz vorzugsweise mit zwei Deckeln durch den Roboter 160 verschlossen.

Bezugszeichen

| | |
|---|---|
| 10 | Analysevorrichtung |
| 20 | Calcinator |
| 30 | Produktentnahme |
| 40 | Produktlager |
| 50 | Probenentnahme |
| 60 | Mühle |
| 70 | Waage |
| 80 | Mischvorrichtung |
| 90 | Laugenvorrat |
| 100 | isothermes Kalorimeter |
| 110 | Analyseelektronik |
| 120 | Steuerelektronik |
| 130 | Laugenzugabevorrichtung |
| 140 | Probenbehältervorrat |
| 150 | Lager- und Temperierbereich |
| 160 | Roboter |
| 170 | Probenzuführung |

**Patentansprüche**

1. Regelungsverfahren zur Regelung eines Herstellungsverfahrens für calcinierte Tone mit einem Calcinator (20), wobei das Regelungsverfahren die folgenden Schritte aufweist:

   a) Erfassen wenigstens einer Temperatur im Calcinator (20) und optional einer ersten mittleren Verweilzeit des Tones im Calcinator (20),
   b) Entnehmen wenigstens einer Probe des calcinierten Tones,
   c) Herstellen einer reproduzierbaren Größenverteilung der Probe,
   d) Einstellen der Probenmasse auf eine vordefinierte Probenmasse,
   e) Temperieren der Probe auf eine erste Messtemperatur und einer Lauge auf eine erste Messtemperatur,
   f) Vermengen der Probe mit einer Lauge,
   g) Zeitliches Erfassen der von der Probe-Laugen-Mischung erzeugten Energie bei konstanter erster Messtemperatur für einen ersten Zeitraum,
   h) Quantitatives Auswerten des in Schritt g) erfassten Zeit-Energie-Verlaufs für die erste exotherme Reaktion und Ermitteln der durch die Probe freigesetzten Energiemenge für die erste exotherme Reaktion,
   i) Korrelation der in Schritt h) erfassten Energiemenge mit der in Schritt a) erfassten Temperatur und Verweilzeit sowie Vergleich mit vorher erfassten Wertekombinationen aus Energiemenge, Temperatur und Verweilzeit,
   j) Aktives Regeln der Temperatur und/oder der Verweilzeit im Calcinator (20) in Richtung der Erhöhung der für eine weitere Probe zu erwartende Energiemenge,

   wobei das quantitative Auswerten des in Schritt g) erfassten Zeit-Energie-Verlaufs für die erste exotherme Reaktion für den Zeitraum von der ersten Minute bis 120. Minute erfolgt.

2. Regelungsverfahren nach Anspruch 1, **dadurch gekennzeichnet, dass** das Herstellen einer reproduzierbaren Größenverteilung der Probe in Schritt c) durch Mahlen erfolgt.

3. Regelungsverfahren nach einem der vorstehenden Ansprüche, **dadurch gekennzeichnet, dass** als Lauge in Schritt f) eine Alkalihydroxidlösung mit einem pH zwischen 9 und 15, bevorzugt zwischen 10,25 und 15, weiter bevorzugt zwischen 12 und 14,6, ausgewählt wird.

4. Regelungsverfahren nach Anspruch 3, **dadurch gekennzeichnet, dass** die Zugabemasse der Lauge dem 0.5fachen bis 5fachen, bevorzugt den 2fachen bis 3fachen, der Masse der Probe entspricht.

5. Regelungsverfahren nach einem der Ansprüche 1 bis 2, **dadurch gekennzeichnet, dass** als Lauge in Schritt f) ein Laugenbildner und Wasser zugegeben werden, wobei der Laugenbildner und das Wasser in Reaktion miteinander eine Lösung mit einem pH zwischen 9 und 15, bevorzugt zwischen 10,25 und 15, weiter bevorzugt zwischen 12 und 14,6, erzeugen.

6. Regelungsverfahren nach Anspruch 5, **dadurch gekennzeichnet, dass** der Laugenbildner ausgewählt ist aus der Gruppe umfassend Alkalihydroxid, Alkalioxid, Erdalkalihydroxid, Erdalkalioxid sowie Stoffe, Gemische oder Zusammensetzungen, die diese enthalten.

7. Regelungsverfahren nach einem der vorstehenden Ansprüche, **dadurch gekennzeichnet, dass** die Schritte c) bis g) automatisiert in einer auf die erste Messtemperatur klimatisierten Umgebung erfolgen.

8. Regelungsverfahren nach einem der vorstehenden Ansprüche, **dadurch gekennzeichnet, dass** die Probenmasse in Schritt d) auf wenigstens 2 % genau, bevorzugt wenigstens 0,5 % genau, weiter bevorzugt wenigstens 0,1 % genau, besonders bevorzugt auf wenigstens 0,02 % genau, auf die vorgegebene Probenmasse eingestellt wird.

9. Regelungsverfahren nach einem der vorstehenden Ansprüche, **dadurch gekennzeichnet, dass** das quantitative Auswerten des in Schritt g) erfassten Zeit-Energie-Verlaufs für die erste exotherme Reaktion für den Zeitraum von zweiten Minute bis zu 70. Minuten erfolgt.

10. Regelungsverfahren nach einem der vorstehenden Ansprüche, **dadurch gekennzeichnet, dass** die erste Messtemperatur im Bereich von 20 °C bis 40 °C gewählt wird.

11. Regelungsverfahren nach einem der vorstehenden Ansprüche, **dadurch gekennzeichnet, dass** die vordefinierte Probenmasse zwischen 1 g und 200 g, bevorzugt zwischen 2 g und 20 g, ausgewählt wird.

12. Regelungsverfahren nach einem der vorstehenden Ansprüche, **dadurch gekennzeichnet, dass** in Schritt a) zusätzlich erfasst wird, aus welchen Eduktchargen in welchem Mischungsverhältnis der Ton der Calcination zugeführt wird, wobei in Schritt i) zusätzlich die Information aus welchen Eduktchargen in welchem Mischungsverhältnis der Ton der Calcination zugeführt wurde, verwendet wird, wobei zur Regelung in Schritt j) zusätzlich die Auswahl und das Mischungsverhältnis zwischen den Eduktchargen berücksichtigt wird.

13. Regelungsverfahren nach Anspruch 12, **dadurch gekennzeichnet, dass** zusätzlich zur Charakterisierung jeder Produktcharge das folgende Chargencharakterisierungsverfahren mit den folgenden Schritten durchgeführt wird:

A) Erwärmen einer Chargenprobe auf eine Chargentemperatur von 600 °C bis 1000 °C, bevorzugt 600 °C bis 950 °C für eine Chargenzeit von 1 s bis 60 min, bevorzugt von 30 s bis 20 min, besonders bevorzugt von 30 s bis 5 min,
B) Herstellen einer reproduzierbaren Größenverteilung der Chargenprobe,
C) Einstellen der Chargenprobenmasse auf eine vordefinierte Probenmasse,
D) Temperieren der Chargenprobe auf eine erste Messtemperatur,
E) Vermengen der Chargenprobe mit einer Lauge,
F) Zeitliches Erfassen der von der Chargenprobe-Laugen-Mischung erzeugten Energie bei konstanter erster Messtemperatur für einen ersten Zeitraum,
G) Quantitatives Auswerten des in Schritt F) erfassten Zeit-Energie-Verlaufs für die erste exotherme Reaktion und Ermitteln der durch die Chargenprobe freigesetzten Energiemenge für die erste exotherme Reaktion.

14. Regelungsverfahren nach Anspruch 13, **dadurch gekennzeichnet, dass** das in Schritt A) für alle Chargenproben eine identische erste Chargentemperatur und eine identische erste Chargenzeit gewählt wird.

15. Regelungsverfahren nach einem der Ansprüche 13 bis 14, **dadurch gekennzeichnet, dass** das Chargencharakterisierungsverfahren mit einer zweiten Chargentemperatur und eine zweiten Chargenzeit wiederholt wird.

**Claims**

1. A control process for controlling a production process for calcined clays with a calciner (20), the control process comprising the following steps:

a) capture of at least one temperature in the calciner (20) and optionally of a first mean residence time of the clay in the calciner (20),
b) taking of at least one sample of the calcined clay,
c) production of a reproducible size distribution of the sample,

d) adjustment of the sample mass to a predefined sample mass,

e) conditioning of the sample to a first measuring temperature and of an alkali to a first measuring temperature,

f) combining of the sample with an alkali,

g) temporal capture of the energy generated by the sample-alkali mixture at constant first measuring temperature for a first period,

h) quantitative evaluation of the time-energy profile captured in step g) for the first exothermic reaction and determination of the amount of energy released by the sample for the first exothermic reaction,

i) correlation of the amount of energy captured in step h) with the temperature and residence time captured in step a), and comparison with previously captured combinations of amount of energy, temperature and residence time values,

j) active control of the temperature and/or of the residence time in the calciner (20) in the direction of increasing the amount of energy anticipated for a further sample,

where the quantitative evaluation of the time-energy profile captured in step g) for the first exothermic reaction takes place for the period from the first minute to minute 120.

2. The control process as claimed in claim 1, **characterized in that** the production of a reproducible size distribution of the sample in step c) takes place by grinding.

3. The control process as claimed in any of the preceding claims, **characterized in that** the alkali selected in step f) is an alkali metal hydroxide solution having a pH of between 9 and 15, preferably between 10.25 and 15, more preferably between 12 and 14.6.

4. The control process as claimed in claim 3, **characterized in that** the mass of added alkali is 0.5 times to 5 times, preferably 2 times to 3 times, the mass of the sample.

5. The control process as claimed in either of claims 1 and 2, **characterized in that** the alkali added in step f) comprises an alkali former and water, where the alkali former and the water in reaction with one another generate a solution having a pH of between 9 and 15, preferably between 10.25 and 15, more preferably between 12 and 14.6.

6. The control process as claimed in claim 5, **characterized in that** the alkali former is selected from the group encompassing alkali metal hydroxide, alkali metal oxide, alkaline earth metal hydroxide, alkaline earth metal oxide, and substances, mixtures or compositions comprising them.

7. The control process as claimed in any of the preceding claims, **characterized in that** steps c) to g) take place automatically in an environment acclimatized to the first measuring temperature.

8. The control process as claimed in any of the preceding claims, **characterized in that** the sample mass in step d) is adjusted to the mandated sample mass to an accuracy of at least 2%, preferably at least 0.5%, more preferably at least 0.1%, very preferably to at least 0.02%.

9. The control process as claimed in any of the preceding claims, **characterized in that** the quantitative evaluation of the time-energy profile captured in step g) for the first exothermic reaction takes place for the period from the second minute up to minute 70.

10. The control process as claimed in any of the preceding claims, **characterized in that** the first measuring temperature is selected in the range from 20° C to 40° C.

11. The control process as claimed in any of the preceding claims, **characterized in that** the predefined sample mass selected is between 1 g and 200 g, preferably between 2 g and 20 g.

12. The control process as claimed in any of the preceding claims, **characterized in that** step a) additionally captures the reactant batches from which and the mixing ratio in which the clay is supplied to the calcination, with the information as to the reactant batches from which and the mixing ratio in which the clay has been supplied to the calcination being used additionally in step i), with control in step j) additionally considering the selection and the mixing ratio between the reactant batches.

13. The control process as claimed in claim 12, **characterized in that** additionally to the characterization of each product batch, the following batch characterization process is carried out, with the following steps:

A) heating of a batch sample to a batch temperature of 600° C to 1000° C, preferably 600° C to 950° C, for a batch time of 1 s to 60 min, preferably of 30 s to 20 min, more preferably of 30 s to 5 min,

B) production of a reproducible size distribution of the batch sample,

C) adjustment of the batch sample mass to a predefined sample mass,

D) conditioning of the batch sample to a first measuring temperature,

E) combination of the batch sample with an alkali,

F) temporal capture of the energy generated by the batch sample-alkali mixture at constant first measuring temperature for a first period,

G) quantitative evaluation of the time-energy profile captured in step F) for the first exothermic reaction, and determination of the amount of energy released by the batch sample for the first exothermic reaction.

14. The control process as claimed in claim 13, **characterized in that** an identical first batch temperature and an identical first batch time are selected for all batch samples in step A).

15. The control process as claimed in either of claims 13 and 14, **characterized in that** the batch characterization process is repeated with a second batch temperature and a second batch time.

## Revendications

1. Un processus de contrôle pour contrôler un processus de production d'argiles calcinées avec un calcinateur (20), le processus de contrôle comprenant les étapes suivantes :

 a) la saisie d'au moins une température dans le calcinateur (20) et, éventuellement, d'un premier temps de séjour moyen de l'argile dans le calcinateur (20),

 b) prélèvement d'au moins un échantillon de l'argile calcinée,

 c) la production d'une distribution de taille reproductible de l'échantillon,

 d) ajustement de la masse de l'échantillon à une masse d'échantillon prédéfinie,

 e) conditionnement de l'échantillon à une première température de mesure et d'un alcali à une première température de mesure,

 f) combinaison de l'échantillon avec un alcali,

 g) la capture temporelle de l'énergie générée par le mélange échantillon-alcali à une première température de mesure constante pendant une première période,

 h) évaluation quantitative du profil temps-énergie saisi à l'étape g) pour la première réaction exothermique et détermination de la quantité d'énergie libérée par l'échantillon pour la première réaction exothermique,

 i) corrélation de la quantité d'énergie capturée à l'étape h) avec la température et le temps de séjour capturés à l'étape a), et comparaison avec des combinaisons de valeurs de quantité d'énergie, de température et de temps de séjour capturées précédemment,

 j) contrôle actif de la température et/ou du temps de séjour dans le four de calcination (20) dans le sens d'une augmentation de la quantité d'énergie attendue pour un autre échantillon,

où l'évaluation quantitative du profil temps-énergie saisi à l'étape g) pour la première réaction exothermique a lieu pour la période allant de la première minute à la minute 120.

2. Procédé de contrôle selon la revendication 1, **caractérisé par le fait que** la production d'une distribution de taille reproductible de l'échantillon à l'étape c) se fait par broyage.

3. Procédé de contrôle selon l'une quelconque des revendications précédentes, **caractérisé par le fait que** l'alcali sélectionné à l'étape f) est une solution d'hydroxyde de métal alcalin ayant un pH compris entre 9 et 15, de préférence entre 10,25 et 15, plus préférentiellement entre 12 et 14,6.

4. Procédé de contrôle selon la revendication 3, **caractérisé par le fait que la** masse d'alcali ajoutée est de 0,5 fois à 5 fois, de préférence de 2 fois à 3 fois, la masse de l'échantillon.

5. Procédé de contrôle selon l'une des revendications 1 et 2, **caractérisé par le fait que** l'alcali ajouté à l'étape f) comprend un alcali formateur et de l'eau, où l'alcali formateur et l'eau en réaction l'un avec l'autre génèrent une solution ayant un pH compris entre 9 et 15, de préférence entre 10,25 et 15, plus préférentiellement entre 12 et 14,6.

6. Procédé de contrôle selon la revendication 5, **caractérisé par le fait que** l'agent alcalin est choisi dans le groupe comprenant l'hydroxyde de métal alcalin, l'oxyde de métal alcalin, l'hydroxyde de métal alcalino-terreux, l'oxyde de métal alcalino-terreux, et les substances, mélanges ou compositions les comprenant.

7. Le procédé de contrôle selon l'une quelconque des revendications précédentes, **caractérisé par le fait que** les étapes c) à g) se déroulent automatiquement dans un environnement acclimaté à la première température de mesure.

8. Procédé de contrôle selon l'une quelconque des revendications précédentes, **caractérisé par le fait que** la masse de l'échantillon à l'étape d) est ajustée à la masse de l'échantillon prescrite avec une précision d'au moins 2 %, de préférence d'au moins 0,5 %, de préférence d'au moins 0,1 %, de préférence encore d'au moins 0,02 %.

9. Procédé de contrôle selon l'une quelconque des revendications précédentes, **caractérisé par le fait**

que l'évaluation quantitative du profil temps-énergie saisi à l'étape g) pour la première réaction exothermique a lieu pour la période allant de la deuxième minute à la minute 70.

10. Procédé de contrôle selon l'une des revendications précédentes, **caractérisé par le fait que** la première température de mesure est choisie dans la plage de 20° C à 40° C.

11. Procédé de contrôle selon l'une quelconque des revendications précédentes, **caractérisé par le fait que** la masse d'échantillon prédéfinie sélectionnée est comprise entre 1 g et 200 g, de préférence entre 2 g et 20 g.

12. Le procédé de contrôle selon l'une quelconque des revendications précédentes, **caractérisé en ce que l'**étape a) saisit en outre les lots de réactifs à partir desquels et le rapport de mélange dans lequel l'argile est fournie à la calcination, les informations concernant les lots de réactifs à partir desquels et le rapport de mélange dans lequel l'argile a été fournie à la calcination étant utilisés en outre à l'étape i), le contrôle à l'étape j) considérant en outre la sélection et le rapport de mélange entre les lots de réactifs.

13. Procédé de contrôle selon la revendication 12, **caractérisé par le fait que** en plus de la caractérisation de chaque lot de produits, le processus suivant de caractérisation des lots est effectué, avec les étapes suivantes :

   A) chauffage d'un échantillon de lot à une température de lot comprise entre 600° C et 1000° C, de préférence entre 600° C et 950° C, pendant une durée de lot comprise entre 1 s et 60 min, de préférence entre 30 s et 20 min, plus préférentiellement entre 30 s et 5 min,
   B) la production d'une distribution de taille reproductible de l'échantillon du lot,
   C) l'ajustement de la masse de l'échantillon du lot à une masse d'échantillon prédéfinie,
   D) conditionnement de l'échantillon de lot à une première température de mesure,
   E) combinaison de l'échantillon de lot avec un alcali,
   F) la capture temporelle de l'énergie générée par le mélange échantillon discontinu-alcali à une première température de mesure constante pendant une première période,
   G) évaluation quantitative du profil temps-énergie saisi à l'étape F) pour la première réaction exothermique et détermination de la quantité d'énergie libérée par l'échantillon de lot pour la première réaction exothermique.

14. Procédé de contrôle selon la revendication 13, **ca-**ractérisé par le fait qu'**une première température de lot identique et une première durée de lot identique sont sélectionnées pour tous les échantillons de lot à l'étape A).

15. Procédé de contrôle selon l'une des revendications 13 et 14, **caractérisé par le fait que** le processus de caractérisation du lot est répété avec une deuxième température de lot et une deuxième durée de lot.

Fig. 1

Fig. 2

Fig. 3

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 3218320 B1
- US 9458059 B2
- DE 102011014498 B4
- DE 102008020600 B4
- US 2012160135 A1
- EP 3615489 A2
- WO 2015039198 A1
- WO 2016041717 A1
- WO 2016096911 A1
- WO 0132581 A1

**In der Beschreibung aufgeführte Nicht-Patentliteratur**

- Development of a new rapid, relevant and reliable (R3) test method to evaluate the puzzolanic reactivity of calcined kaolinitic clay. **F. AVET et al.** Cement and Concrete Research. Elsevier, 2016, vol. 85, 1-11
- **ROGER S. ALMENARES et al.** Industrial calcination of kaolinitic clays to make reactive pozzolans. *CASE STUDIES IN CONSTRUCTION MATERIALS,* 01. Juni 2017, vol. 6, ISSN 2214-5095, 225-232